# EUROPEAN PATENT APPLICATION

(11) **EP 4 494 571 A1**
(43) Date of publication of application: **22.01.2025**
(21) Application number: 22936442.7
(22) Date of filing: 04.04.2022
(51) Int. Cl.: A61B 17/00

(54) **INDWELLING MEDICAL OBJECT**

(71) Applicant: Terumo Kabushiki Kaisha, Tokyo 151-0072 (JP)
(72) Inventor: ANZAI, Takao, Ashigarakami-gun, Kanagawa 259-0151 (JP)
(74) Representative: Prüfer & Partner mbB Patentanwälte · Rechtsanwälte
(86) International application number: PCT/JP2022/017024
(87) International publication number: WO 2023/195039

(57) **Abstract**

Provided is a medical indwelling object capable of shortening a period until complete occlusion of an aneurysm 100 and endothelialization of an entrance part of the aneurysm 100. A flow diverter stent 11 as a medical indwelling object is configured to be indwelled in a biological lumen. The flow diverter stent has a main body part 20 and a filament 40 made of a non-biodegradable material combined with the main body part. The filament is composed of a porous body having a large number of pores 41 having a diameter in a range of 20 to 40 um, the pores all having a diameter falling within ± 10%, arranged side by side, and each of the pores being communicated with at least three other of the pores.

## Description

### Technical Field

The present invention generally relates to a medical indwelling object indwelled in a biological lumen.

### Background Art

For vascular diseases such as cerebral aneurysm, pulmonary aneurysm, and renal aneurysm, a procedure for preventing burst of the aneurysm by filling and occluding a metal coil is widely performed. On the other hand, as a new procedure, a method is performed in which a tubular body (flow diverter stent) obtained by interweaving a metal wire (particularly, nickel titanium) is indwelled in a parent blood vessel of the aneurysm to block and stagnate the blood flow into the aneurysm, and the blood in the aneurysm is thrombosed and organized. In addition, a method of interweaving a metal wire into a basket shape, indwelling the basket in an aneurysm, and similarly promoting organization is also performed.

A vascular graft according to Patent Literature 1 below is configured to occlude a blood vessel system. This vascular graft creates a capillary effect inside it that allows blood to be carried through the vascular graft and coagulate within the vascular graft.

### Citation List

### Patent Literature

Patent Literature 1: JP 2018-502657 T

### Summary of Invention

### Technical Problem

However, the vascular graft of the Patent Literature 1 merely considers that blood components are absorbed and cells enter, and may merely promote thrombus formation and an inflammatory reaction.

When a medical indwelling object is indwelled in a blood vessel, a patient indwelled with the indwelling product needs to take an antiplatelet drug or an anticoagulant for a long period of time. This is because there is a risk that the surface of the indwelling object becomes a starting point of thrombus formation. Therefore, early acquisition of a state in which the surface of the indwelling object is covered with vascular endothelial cells, that is, early endothelialization is required.

The present invention has been made to solve the problems associated with the prior art, and it is an object of the present invention to provide a medical indwelling object capable of shortening a period until complete occlusion of an aneurysm and endothelialization of an entrance part of the aneurysm.

### Solution to Problem

The present invention for achieving the above object is a medical indwelling object indwelled in a biological lumen, and the medical indwelling object includes a main body part and a filament made of a non-biodegradable material combined with the main body part. The filament is a porous body having a large number of pores in the range of 20 to 40 um in diameter, the pores all having a diameter falling within ± 10%, arranged side by side, and each of the pores being communicated with at least the other three of the pores.

### Advantageous Effects of Invention

According to the medical indwelling object according to the present invention, due to the pore structure of the filament, the inner part of the filament exerts an action including many M1 macrophages (pro-inflammatory reaction) among macrophages in the blood, and as a result, the blood around the filament has a relatively high ratio of M2 macrophages (pro-healing). Such polarization of macrophages shortens the time to complete occlusion of the aneurysm or endothelialization of the entrance part of the aneurysm. In addition,

Since the filament is made of a non-biodegradable material, there is no possibility that the material is decomposed and exfoliated, flows into a blood vessel, and occludes the periphery. Therefore, it is possible to provide a medical indwelling object capable of shortening a period until complete occlusion of the aneurysm and endothelialization of the entrance part of the aneurysm.

### Brief Description of Drawings

Fig. 1 is a view schematically illustrating a state in which a flow diverter stent as a medical indwelling object is indwelled between a parent blood vessel and an aneurysm.
Fig. 2A is a side view illustrating a main part of the flow diverter stent.
Fig. 2B is an end view taken along line 2B-2B in Fig. 2A, schematically illustrating a combination form of the main body part and the filaments.
Fig. 3 is a perspective view schematically illustrating a filament of a flow diverter stent.
Fig. 4 is an enlarged view illustrating a part surrounded by a reference numeral 4 in Fig. 3 and illustrating a pore structure of the filament.
Fig. 5 is a view schematically illustrating a method for manufacturing the filament.
Fig. 6A is an enlarged cross-sectional view schematically illustrating a part of a cross section taken along line 6A-6A in Fig. 5.
Fig. 6B is an enlarged cross-sectional view schematically illustrating a part of a cross section taken along line 6B-6B in Fig. 5.
Fig. 7 is a view schematically illustrating a state in which an embolic material as a medical indwelling object is indwelled in an aneurysm.
Fig. 8 is an enlarged view illustrating a main part of the embolic material.

### Description of Embodiments

Hereinafter, embodiments of the present invention and modifications thereof is described with reference to the accompanying drawings. The following description does not limit the technical scope or the significance of each term used in the claims. Furthermore, dimensional ratios of the drawings are exaggerated for illustration purpose and may differ from actual ratios.

### (First Embodiment (Flow diverter stent 11))

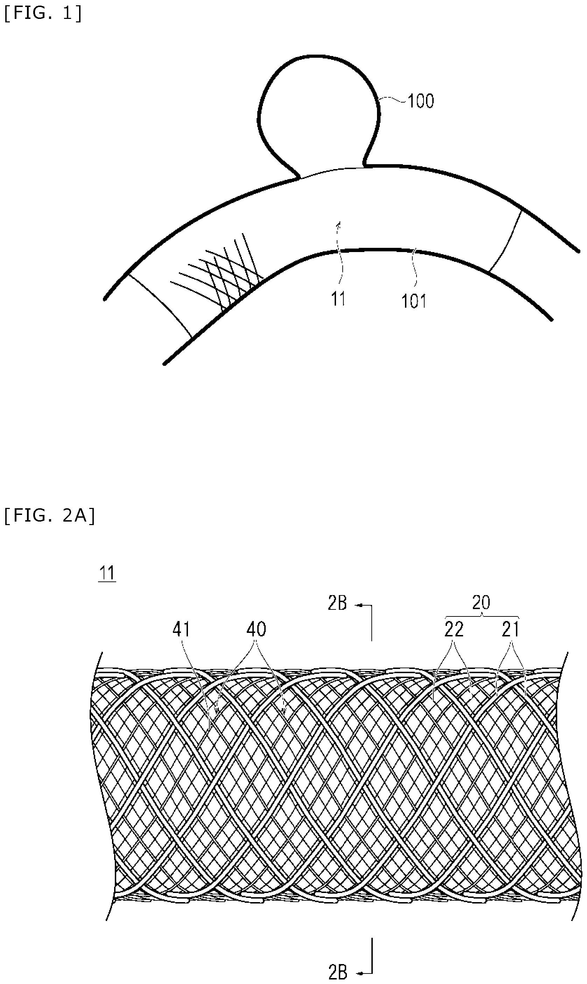

In the first embodiment, the medical indwelling object of the present invention is applied to the flow diverter stent 11 (stent 11) that blocks the blood flow between the parent blood vessel 101 and the aneurysm 100. The parent blood vessel 101 and the aneurysm 100 correspond to the biological lumen. Fig. 1 is a view schematically illustrating a state in which a flow diverter stent 11 as a medical indwelling object is indwelled between a parent blood vessel 101 and an aneurysm 100. In Fig. 2A, the gap part is greatly exaggerated in order to easily grasp the mesh shape of the flow diverter stent 11.

As illustrated in Fig. 1, the flow diverter stent 11 is indwelled between the parent blood vessel 101 and the aneurysm 100, and is used to block the blood flow between the parent blood vessel 101 and the aneurysm 100. The flow diverter stent 11 is a stent having self-expandability. The flow diverter stent 11 is inserted and indwelled to a target site of the parent blood vessel 101 by a self-expanding stent delivery system (not illustrated).

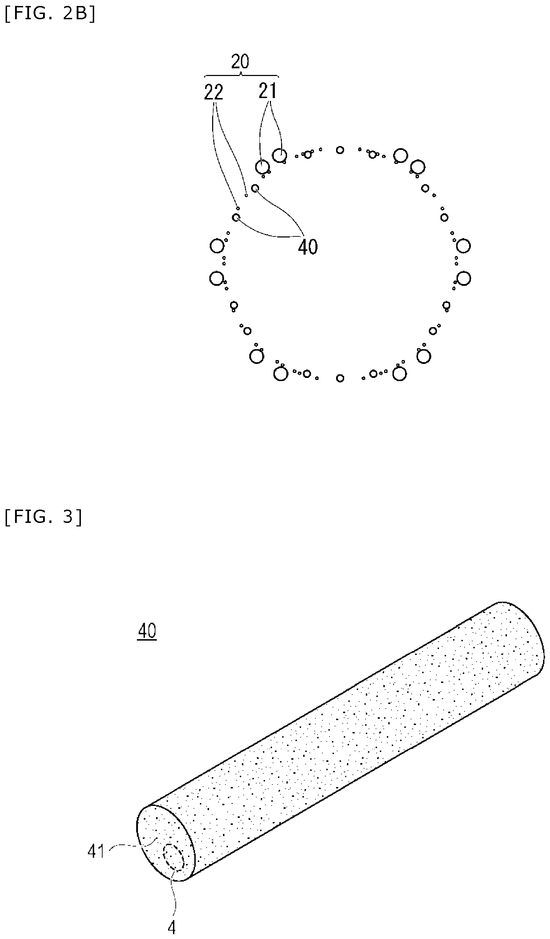

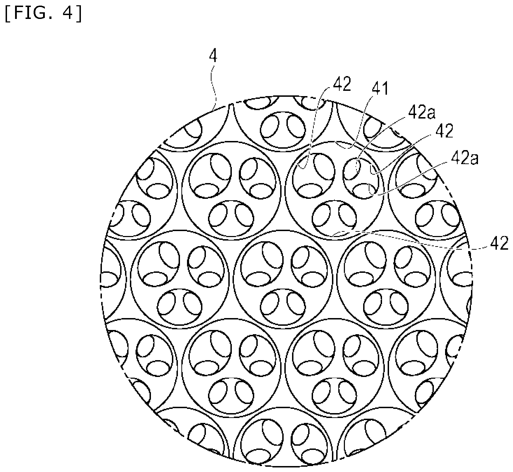

Fig. 2A is a side view illustrating a main part of the flow diverter stent 11, and Fig. 2B is an end view taken along line 2B-2B in Fig. 2A, schematically illustrating a combination form of the main body part 20 and the filaments 40. Fig. 3 is a perspective view schematically illustrating a filament 40 of the flow diverter stent 11, and Fig. 4 is an enlarged view of a part surrounded by reference numeral 4 in Fig. 3, illustrating a pore structure of the filament 40.

As illustrated in Figs. 2A, 3, and 4, the flow diverter stent 11 is an example of one of the medical indwelling objects indwelled in the biological lumen, and includes the main body part 20 and filaments 40 made of a non-biodegradable material that is combined with the main body part 20.

Each filament 40 is composed of a porous body having a large number of pores 41 arranged side by side. The pores 41 may have an inner diameter in the range of 20 to 40 um, and the pores 41 have a substantially uniform inner diameter, meaning any variation in the diameter of a pore falls within ± 10%. Each of the pores 41 communicates with at least three other pores 41. Hereinafter, the configuration of the flow diverter stent 11 is described in detail.

### (Main body part 20)

As illustrated in Fig. 2A, the main body part 20 is a stent having self-expandability, and is composed of a first wire(s) 21 made of metal. The first wire 21 is braided in a mesh shape to form tubular stent struts. The metal used for the first wire 21 is preferably a biocompatible metal, and examples thereof include stainless steel, tantalum or a tantalum alloy, platinum or a platinum alloy, gold or a gold alloy, a cobalt base alloy such as a cobalt-chromium alloy, and a nickel-titanium alloy. Among them, a nickel-titanium alloy, which is a material having superelasticity, is most preferable in order to achieve self-expandability. In addition, precious metal plating (gold, platinum) for imparting X-ray contrast property may be applied after the shape of the stent is prepared. The diameter of the first wire 21 is not particularly limited, and is, for example, 100 um (micrometer). The tubular body composed of the first wire 21 of a relatively thick metal wire constitutes a framework as a self-expanding stent.

In the main body part 20, second wires 22 made of metal having a diameter smaller than that of the first wire 21 are combined with the first wire 21. The second wires 22 are braided at a finer pitch than the mesh of the first wire 21 so as to close the gap between the first wires 21, and is arranged inside the tubular mesh of the first wire 21. With this configuration, minute gaps between the first wire(s) 21 are blocked or closed by the second wires 22, thereby blocking (minimizing) blood flow from the parent blood vessel 101 side to the aneurysm 100 side. The metal used for the second wires 22 may be the same metal as the metal used for the first wire 21. The diameter of the second wires 22 is not particularly limited, and is, for example, 25 to 30 um. The tubular body composed of the first wire 21 and the tubular body composed of the second wires 22 are fixed by a third wire (not illustrated). As the third wire, for example, a metal having X-ray contrast property is used, and tungsten is particularly suitable. A plurality of third wires are provided so that a tubular body composed of the first wire 21 and a tubular body composed of the second wire 22 are sewn together in the axial direction.

### (Filaments 40)

The filaments 40 can be combined on the inner surface side, the outer surface side of the main body part 20, or within the main body part 20. As illustrated in Figs. 2A and 2B, in the flow diverter stent 11 of the first embodiment, the filaments 40 are incorporated (interwoven) in the tubular body composed of the second wires 22 in a manner replacing a plurality of the second wires.

As such, the filaments 40 may be combined into the main body part 20. The phrase "combined into or within the main body part 20" refers to a form in which the second wires 22 and the filaments 40 are substantially on/in the same plane when viewed in a cross section, by weaving the second wires 22 into a mesh configuration and weaving the filaments 40 at the same time, or by later inserting the filaments 40 into the opening parts of the main body part 20.

In the main body part 20, as described above, the second wires 22 are further combined with the first wire 21. Therefore, when the filaments 40 are combined in the main body part 20, a part of the second wire 22 may be replaced with the filaments 40. The replacing ratio can be appropriately set.

As a modification, the filaments 40 may be braided in a mesh shape separately from the main body part 20 and housed in the lumen of the main body part 20. The mesh-shaped filaments 40 are fixed to the main body part 20 at an appropriate position so as not to hinder the self-expandability of the main body part 20. The fixing method is not limited, but for example, the filaments 40 may be wound around and fixed to the first wire 21 of the main body part 20 by a tungsten wire having X-ray contrast property.

In addition, as a second modification, the filaments 40 formed in a mesh shape may be combined with the outer surface side of the main body part 20. In this embodiment, the filaments 40 are braided in a mesh shape separately from the main body part 20, and the resulting mesh-shaped knitted filaments 40 are positioned to cover the outer peripheral surface of the main body part 20. The mesh-shaped filaments 40 are fixed to the main body part 20 at an appropriate position so as not to hinder the self-expandability of the main body part 20. The fixing method is not limited, but the filaments 40 may be wound around and fixed to the first wire 21 of the main body part 20 by the tungsten wire having the X-ray contrast property as described above.

The material constituting the filaments 40 is preferably a non-biodegradable material and a flexible material. A metal or resin material can be applied as a constituent material of the filaments 40, but a resin material is preferable from the viewpoint of manufacturing a pore structure to be described later. The material of the resin material needs to be a material that is not decomposed in the living body. Specifically, as the resin composition constituting the filament 40, polyolefin, polyethylene, a polyolefin elastomer, an ethylene-octene copolymer, polyethylene terephthalate (PET), nylon, an amide-based polymer, a block copolymer, a polyether block amide copolymer (for example, PEBAX (registered trademark) manufactured by Arkema K.K.), and polypropylene are suitably used.

The diameter of the filaments 40 is not particularly limited, but because the filaments 40 are porous bodies in which each pore 41 is preferably connected to at least three other pores 41, it is preferably that the diameter of the filaments 40 is 60 to 120 um in relation to the inner diameter (20 to 40 µm) of the pores 41.

As shown in Figs. 3 and 4, each of the filaments 40 is a porous body having a large number of pores 41 having an inner diameter in a range of 20 to 40 um, the pores 41 having a substantially uniform inner diameter such that any variation in the diameters of all pores fall within ± 10%, arranged side by side in an array, and each of the pores 41 being in communication with at least the other three pores 41. Fig. 4 illustrates an axis-orthogonal cross section of the filaments 40 of the porous body having the above-described pore structure. The circle indicated by reference numeral 41 indicates a large number of pores 41 having a diameter in the range of 20 to 40 µm, and any variation in the diameters of all the pores 41 falling within ± 10%. Each of the pores 41 is arranged side by side in an array in a close-packed state. Three ellipses indicated by reference numeral 42 in one pore 41 indicate communication pores 42 in which adjacent pores 41 are in contact with each other and the adjacent pores 41 communicate with each other at a contact point. Furthermore, two ellipses denoted by reference numeral 42a in one communication pore 42 indicate a communication pore 42a that can be seen further on the back side through the communication pore 42.

In order to obtain a uniform pore structure, it is preferable that all diameters of the pores 41 fall within ± 10% of each other.

Each of the pores 41 may communicate with three other pores 41 to twelve other pores 41. Most preferably, each pore 41 communicates with six or twelve other pores 41, except for the pores 41 exposed on the outer surface of the filaments 40. In the present embodiment, as shown in Fig. 4, the pores communicate with three pores on the back side from the center, similarly communicate with three pores in front, and further communicate with six pores surrounding the side surface, so that the pores communicate with a total of twelve pores. In the present embodiment, substantially all the pores 41 communicate with at least the other three pores 41, but the number of the adjacent other pores 41 is limited for the pores 41 located on the outer surface of the filaments 40, and thus a case where the number of the other pores communicating with each other is smaller than three is also acceptable.

The tissue repair process after indwelling of the flow diverter stent 11 is similar to the healing process of a skin wound, passing through the inflammatory phase → the proliferative phase → the maturation phase, and then transformation into neointima, completing the healing process. The porous filaments 40 of this embodiment having the above-described pore structure has the effect of shortening the period required for tissue repair (neointima formation) through the inflammatory phase → the proliferative phase → the maturation phase. The mechanism behind this is thought to be that the pore structure of the filaments 40 causes the inner part of the filaments 40 to capture many M1 macrophages (pro-inflammatory reaction) among the macrophages in the blood, so that the macrophages around the filaments 40 (around the flow diverter stent 11) are polarized into a state of a high ratio of M2 macrophages (pro-healing). M1 macrophages produce pro-inflammatory cytokines and M2 macrophages produce antiinflammatory cytokines. It is considered that complete occlusion of the aneurysm 100, healing of the entrance part of the aneurysm 100, and endothelialization are completed early by the polarization of macrophages. Such an action is particularly remarkable when the diameter of the pores 41 is in the range 20 to 40 µm.

At present, the detailed mechanism by which the polarization of macrophages occurs in a specific pore structure has not been clarified clearly.

In the flow diverter stent 11 of the present embodiment configured as described above, the pore structure of the filament 40 promotes not only enhancement of the inflammatory reaction but also promotion of the healing reaction with respect to the occlusion of the aneurysm 100. Therefore, it is possible to reduce the possibility that the period until complete occlusion due to continuation of the inflammatory reaction is prolonged or endothelialization becomes insufficient.

In addition, since the filaments 40 have both a healing promoting function and flexibility, it can be indwelled in the parent blood vessel 101 without impairing the operability of the flow diverter stent 11 and the followability to the blood vessel wall. After indwelling, the filaments 40 of the porous structure can promote a healing reaction and achieve early endothelialization. Since the filament 40 is flexible, the self-expandability of the main body part 20 can be favorably maintained.

In addition, since the filament 40 is configured from a non-biodegradable material, a situation in which the material is decomposed and flows into the blood stream to occlude the periphery does not occur.

### (Method for manufacturing filaments 40)

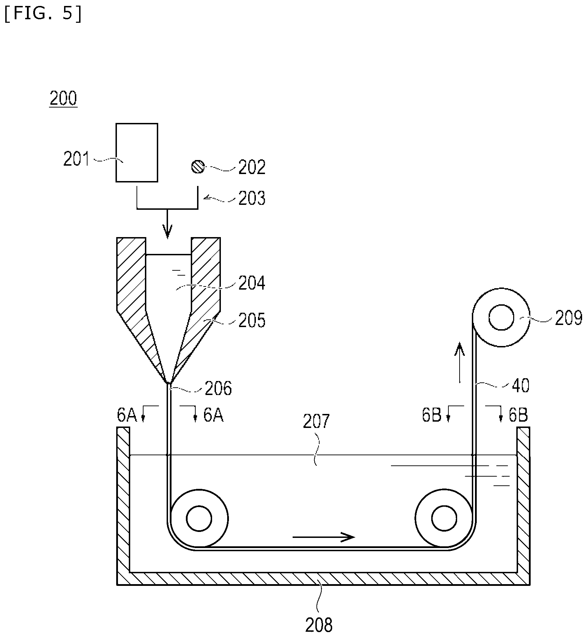

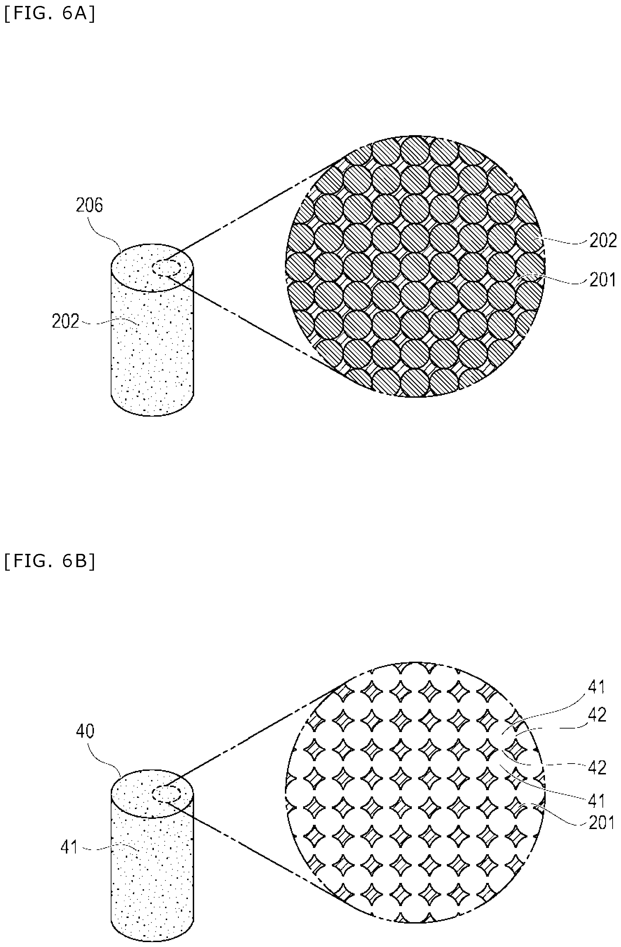

Fig. 5 is a view schematically illustrating a method of manufacturing the filaments 40. Fig. 6A is a cross-sectional view taken along line 6A-6 A in Fig. 5, and Fig. 6B is a cross-sectional view taken along line 6B-6B in Fig. 5. Each of the cross-sectional views schematically illustrates a part of a cross section orthogonal to the longitudinal axis of the filaments 40 in an enlarged manner.

As shown in Fig. 5, a manufacturing machine 200 for a filament 40 includes a supply unit 203 that supplies a raw material solution 201 of a resin composition constituting the filament 40 and a pore-forming material 202 for forming pores 41, a nozzle 205 that discharges a mixed liquid 204 of the raw material solution 201 and the pore-forming material 202 in stringlike, a tank 208 that stores a solvent 207 that allows passage of the raw material yarn 206 discharged from the nozzle 205, and a product roller 209 that winds up the filament 40 having a pore structure.

The resin composition constituting the filament 40 is as described above. It is preferable that the pore-forming material 202 has a perfect spherical shape having a diameter within a range of 20 to 40 µm, and all diameters fall within ± 10%. The pore-forming material 202 preferably has hardness capable of maintaining a perfect spherical shape during molding. The pore-forming material 202 is dissolved in a solvent 207 that does not dissolve the resin composition constituting the filament 40. The pore-forming material 202 is preferably formed of, for example, polymethyl methacrylate or polystyrene, and the solvent 207 used for dissolution is preferably, for example, tetrahydrofuran, chloroform, or the like. The shape of the nozzle 205 outlet and the discharge pressure for discharging the mixed liquid 204 is adjusted so that the pore-forming material 202 mixed in the raw material solution 201 comes into uniform contact with each other.

The tank 208 is filled with a solvent 207 for dissolving only the pore-forming material 202. The inner diameter of the nozzle 205 defines the outer diameter of the filament 40, but the inner diameter of the nozzle 205 is desirably an integral multiple of the outer diameter of the pore-forming material 202 so that the pore-forming material 202 is uniformly disposed in the filament 40.

As illustrated in Fig. 6A, in the raw material yarn 206 discharged from the nozzle 205, the pore-forming materials 202 mixed in the raw material solution 201 are uniformly in contact with each other. While the raw material yarn 206 is moved in the solvent 207 in the tank 208, only the pore-forming material 202 is gradually dissolved and removed from the raw material yarn 206. As illustrated in Fig. 6B, when the raw material yarn 206 is pulled up from the solvent 207 in the tank 208, all the pore-forming materials 202 are dissolved and removed. As a result, the filament 40 of the porous body having the pore structure in which the plurality of pores 41 communicate with each other and are arranged in an array is obtained.

In order to form the pores 41 without gaps as much as possible in the above-described manufacturing process, the cross section of the filament 40 may not be circular, and may be a square or a rectangle in which one side is an integral multiple of the outer diameter of the pore-forming material 202. When the pore-forming material 202 is not disposed on the outer surface of the manufactured filament 40 and the pores 41 are not formed, the filament 40 may be finished by polishing or cutting the surface of the extrusion-molded filament.

### (Effect of flow diverter stent 11)

As described above, the flow diverter stent 11 of the present embodiment is one of medical indwelling objects indwelled in the biological lumen, and includes the main body part 20 and the filaments 40 made of a non-biodegradable material combined with the main body part 20. The filaments 40 are a porous bodies having a large number of pores 41 possessing a diameter in a range of 20 to 40 um, the pores 41 all having a diameter falling within ± 10%, arranged side by side, and each of the pores 41 communicating with at least three of the other pores 41.

According to the stent 11 configured in this manner, the characteristic pore structure of the filaments 40 allows the inner part of each filament 40 capture a large number of M1 macrophages (that promote inflammatory reaction) among macrophages in the blood, and as a result, the blood outside the filaments 40 (around the stent 11) has a high proportion of M2 macrophages (that promote healing). This polarization of macrophages has the effect of shortening the time period required for tissue repair (neointima formation) through the inflammatory phase → the proliferative phase → the maturation phase. As a result, the time period until complete occlusion of the aneurysm 100 or endothelialization of the entrance part of the aneurysm 100 is shortened. Moreover, since the filament 40 is made of a non-biodegradable material, there is no possibility that the material is decomposed and exfoliated, flows out into the blood vessel, and occludes the periphery. Therefore, promoting the healing reaction by controlling the phenotype of macrophages makes it possible to provide a stent 11 that is capable of shortening the time period required to complete occlusion of the aneurysm 100 and endothelialization of the entrance part of the aneurysm 100.

All of the pores 41 preferably have a substantially uniform inner diameter such that any variation in the inner diameter of a pore is within ± 10%. With this configuration, the pore structure of the filaments 40 becomes uniform, and the action of shortening the period required for tissue repair (neointima formation) can be reliably exerted.

The main body part 20 is composed of the first wire 21 made of metal, and the filaments 40 that are combined on the inner surface side or the outer surface side of the main body part 20 or in the main body part 20. With this configuration, since the filaments 40 come into contact with or come close to the inner wall surface of the biological lumen, the action of shortening the period required for tissue repair (neointima formation) can be more reliably realized.

The main body part 20 has a braided body including a first wire 21 made of metal, and the filaments 40 are interwoven into the braided structure of the main body part 20. With this configuration, the skeleton of the stent 11 can be easily configured by the first wire 21 of the metal wire.

In the main body part 20, second wires 22 made of metal having a diameter smaller than that of the first wire 21 is further combined with the first wire 21. With this configuration, the gap between the first wires 21 can be closed by the second wires 22, and the blood flow from the parent blood vessel 101 side to the aneurysm 100 side is blocked. Meanwhile, blood flow is secured in the stent 11. By blocking the aneurysm 100 from the parent blood vessel 101, it is possible to thrombose the aneurysm 100 at an early stage and prevent rupture.

The filaments 40 are interwoven with the second wire 22. With this configuration, the filament 40 can be easily combined in the main body part 20.

The resin composition constituting the filaments 40 is selected from polyolefin, polyethylene, polyolefin elastomer, ethylene-octene copolymer, polyethylene terephthalate, nylon, an amide-based polymer, a block copolymer, a polyether block amide copolymer, and polypropylene. With this configuration, the flexible filaments 40 made of a non-biodegradable material can be obtained. As a result, the stent 11 can be indwelled in the parent blood vessel 101 without impairing the operability of the stent and the followability to the blood vessel wall, and after the indwelling, the filaments 40 of the porous structure promotes a healing reaction and can achieve early endothelialization.

### (Second Embodiment (Embolic material 12))

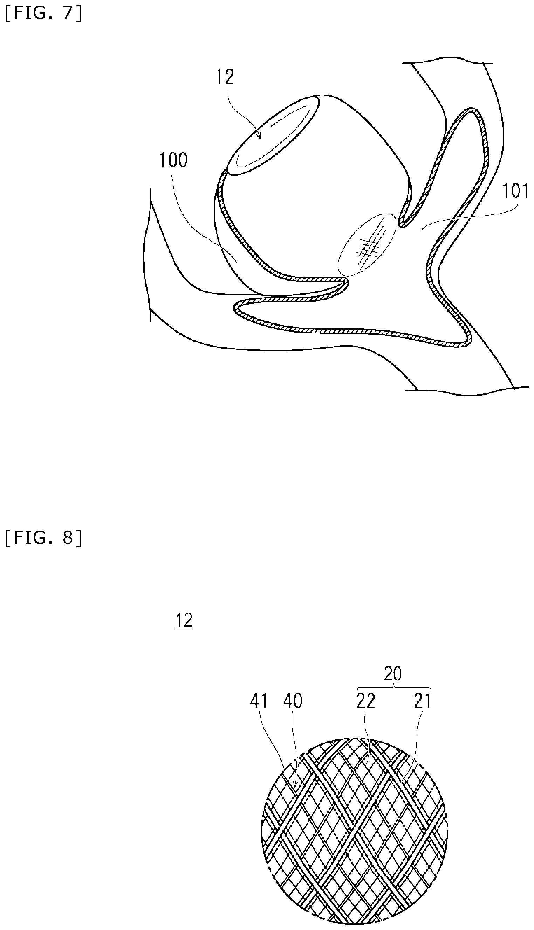

In the second embodiment, the medical indwelling object of the present invention is applied to the embolic material 12 indwelled in the aneurysm 100 in order to close the inflow port from the parent blood vessel 101 to the aneurysm 100. Fig. 7 is a view schematically illustrating a state in which the embolic material 12 as the medical indwelling object is indwelled in the aneurysm 100, and Fig. 8 is an enlarged view illustrating a main part of the embolic material 12.

One known method for treating the cerebrovascular aneurysm 100 involves embolization by a coil. However, in order to pack the coils into the aneurysm 100, it is necessary to arrange as many coils as needed to fill the volume of the aneurysm 100, which increases the operation time. In addition, when the entrance between the aneurysm 100 and the parent blood vessel 101 is large, there is a possibility that the coil disposed in the aneurysm 100 spills over to the parent blood vessel 101. Furthermore, since a small amount of blood flow occurs between the aneurysm 100 and the parent blood vessel 101, it takes a relatively long time to thrombose the aneurysm 100. Since the aneurysm 100 may rupture until it thromboses, it is desirable to block blood flow between the aneurysm 100 and the parent blood vessel 101.

Therefore, the medical indwelling object of the present embodiment is a substantially spherical embolic material 12 that blocks the blood flow between the inside and the outside, is disposed in the aneurysm 100 in a compressed state, self-expands so as to return to the original substantially spherical shape by being released from the compressed state, and is suitable for constituting the embolic material 12 that is indwelled in the aneurysm 100 and used to close the inflow port from the parent blood vessel 101 to the aneurysm 100.

The embolic material 12 is one of medical indwelling objects indwelled in the biological lumen, and includes a main body part 20 and filaments 40 made of a non-biodegradable material combined with the main body part 20.

Like the main body part 20 of the flow diverter stent 11 described above, the main body part 20 is configured by braiding the first wire 21 made of metal, and the filaments 40 are combined in the main body part 20 as a part of the braided wire of the main body part 20.

With this configuration, since the filament 40 comes into contact with or comes close to the inner wall surface of the biological lumen, the action of shortening the period required for tissue repair (neointima formation) can be more reliably exerted.

Similar to the first embodiment, in the main body part 20, the first wire 21 may be further combined with the second wire 22 made of metal having a diameter smaller than that of the first wire 21. With this configuration, the gap between the first wires 21 can be closed by the second wire 22, and the blood flow from the parent blood vessel 101 side to the aneurysm 100 side can be blocked. By blocking the aneurysm 100 from the parent blood vessel 101, it is possible to thrombose the aneurysm 100 at an early stage and prevent rupture.

The filaments 40 are the same as the filaments 40 of the first embodiment (see Fig. 3 and Fig. 4). The filaments 40 are composed of a porous body that includes a large number of pores 41 having a diameter within a range of 20 to 40 um, all the pores 41 having a diameter falling within ± 10%, arranged side by side, and each of the pores 41 excluding the pores exposed on the outer surface of the filament 40 communicates with at least three to twelve other pores 41. Particularly preferably, each of the pores communicates with the other 6 or 12 pores 41 except for the pores near the surface of the filaments 40.

The embolic material 12 of the second embodiment configured as described above has the same action and effect as those of the flow diverter stent 11 of the first embodiment. That is, according to the embolic material 12, the characteristic pore structure of the filaments 40 causes, the inner part of the filaments 40 to capture a large number of M1 macrophages (that promote inflammatory reaction) among macrophages in the blood, and as a result, the blood outside the filament 40 (around the embolic material 12) has a high proportion of M2 macrophages (which promote healing). This polarization of macrophages has the effect of shortening the time period required for tissue repair (neointima formation) through the inflammatory phase → the proliferative phase → the maturation phase. As a result, the period until complete occlusion of the aneurysm 100 or endothelialization of the entrance part of the aneurysm 100 is shortened. Moreover, since the filaments 40 are made of a non-biodegradable material, there is no possibility that the material is decomposed and exfoliated, flows out into the blood vessel, and occludes the periphery. Therefore, it is possible to provide the embolic material 12 capable of shortening the period until complete occlusion of the aneurysm 100 and endothelialization of the entrance part of the aneurysm 100 by promoting the healing reaction by controlling the phenotype of macrophages.

Although the medical indwelling object according to the present invention has been described through the first embodiment and the second embodiment, the present invention is not limited to these cases, and can be appropriately changed based on the description of the claims.

Although the embodiment in which only the filaments 40 of the porous structure are combined with the main body part 20 is described, the present invention is not limited to this case. For example, the filaments 40 and a filament-shaped body made of another resin or metal are twisted to form a twisted wire, and the twisted wire is combined with the main body part 20 to form a medical indwelling object.

Further, it can be modified as follows. As one of the methods for treating the aortic aneurysm 100, stent-graft indwelling technique is known. Stent-graft indwelling technique is a way for supporting and indwelling a graft that reinforces a blood vessel wall from the inside by a stent to prevent rupture of the aneurysm 100. The graft is an artificial blood vessel interwoven with water-repellent fibers. The flow diverter stent 11 of the first embodiment can be used as a stent graft since it can prevent the flow of liquid between the inside and the outside. In this case, since a graft as a fiber layer is unnecessary, a stent graft having a small diameter and high flexibility can be obtained. As a result, the stent graft can also be used in thin blood vessels.

### Reference Signs List

- 11: Flow diverter stent (stent, medical indwelling object)
- 12: Embolic material (medical indwelling object)
- 20: Main body part
- 21: First wire
- 22: Second wire
- 40: Filament
- 41: Pore
- 42: Communication pore
- 42a: Communication pore
- 100: Aneurysm (biological lumen)
- 101: Parent blood vessel (biological lumen)
- 200: Manufacturing machine
- 201: Raw material solution
- 202: Pore-forming material
- 203: Supply unit
- 204: Mixed liquid
- 205: Nozzle
- 206: Raw material yarn
- 207: Solvent that dissolves only pore-forming material
- 208: Tank
- 209: Product roller

## Claims

1. A medical indwelling object configured to be indwelled in a biological lumen, comprising:
a main body part,
a filament made of a non-biodegradable material combined with the main body part,
the filament being a porous body having a plurality of pores each having a diameter in a range of 20 to 40 um, the pores all having a diameter such that any variation in the diameter is within ± 10%, the pores being arranged side by side, and each of the pores being in communication with at least three others of the pores.

2. The medical indwelling object according to claim 1, wherein the main body part is comprised of a first wire made of metal, and
the filament is positioned on an inner surface side of the main body part or an outer surface side of the main body part, or in the main body part.

3. The medical indwelling object according to claim 1, wherein the main body part includes a braided body including the first wire made of metal, and
the filament is interwoven into a braided structure of the main body part.

4. The medical indwelling object according to claim 2 or 3, wherein the main body part is formed by further combining a second wire made of metal having a diameter smaller than that of the first wire with the first wire.

5. The medical indwelling object according to claim 4, wherein the filament is interwoven together with the second wire.

6. The medical indwelling object according to claim 1, wherein a resin composition constituting the filament is selected from polyolefin, polyethylene, polyolefin elastomer, ethylene-octene copolymer, polyethylene terephthalate, nylon, an amide-based polymer, a block copolymer, a polyether block amide copolymer, and polypropylene.

7. The medical indwelling object according to claim 1, wherein the medical indwelling object is a flow diverter stent that blocks blood flow between the parent blood vessel and the aneurysm.

8. The medical indwelling object according to claim 1, wherein the medical indwelling object is an embolic material indwelled in the aneurysm in order to close an inflow port from the parent blood vessel to the aneurysm.
